# EUROPEAN PATENT APPLICATION

(11) **EP 4 813 372 A1**
(43) Date of publication of application: **30.09.2026**
(21) Application number: 25382278.7
(22) Date of filing: 24.03.2025
(51) Int. Cl.: A61K 8/99, A61Q 19/00, A61Q 19/08

(54) **FERMENT EXTRACT OF BACILLUS VELEZENSIS, COSMETIC COMPOSITION COMPRISING SUCH EXTRACT AND USES THEREOF**

(71) Applicant: Provital, S.A., 08210 Barberà del Vallès (ES)
(72) Inventor: MANZANO ALÍAS, David, 08025 BARCELONA (ES); ASO PÉREZ, Miguel, 08191 RUBÍ (ES); ESCUDERO FERNÁNDEZ, Elizabeth, 43840 SALOU (ES)
(74) Representative: Sugrañes, S.L.P.

(57) **Abstract**

The present invention relates to a ferment extract of a strain of *Bacillus velezensis* species deposited in the Spanish Type Culture Collection under accession number CECT 31027, to a method for the cosmetic, non-therapeutic treatment and/or care of the skin comprising the topical administration of an effective amount of a ferment extract, and to uses thereof, in particular to cosmetic uses for improving the appearance of acne-prone skin, reducing the appearance of oil or pores on the skin and/or reversing the effects caused by ageing on the skin. It also relates to a cosmetic composition comprising the ferment extract, and to a cosmetic method for improving the state of the skin, preferably human skin.

## Description

### Technical field of the invention

The present invention relates to the field of compositions for the topical application on the skin in order to improve the state of the skin, preferably human skin.

### Background of the invention

The skin is a large and complex tissue providing a protective interface between an organism and its environment. It is a complex organ that performs numerous vital functions, such as protection, thermoregulation and sensation. One of the main functions is to act as a protective barrier between the outside and the inside of the body, by protecting from external factors such as bacteria, chemicals and temperature. Epidermis forms its external surface and is mainly constituted of multiple layers of specialized epithelial cells named keratinocytes.

Moreover, fibroblasts are specialized cells found within connective tissues, and they play a crucial role in the synthesis and maintenance of the extracellular matrix (ECM). These cells are responsible for producing collagen, elastin, and other structural proteins that provide strength and elasticity to tissues, e.g. skin. Fibroblasts also produce glycosaminoglycans and proteoglycans, which contribute to the hydration and resilience of tissues. In the skin, fibroblasts are primarily located in the dermis, the layer beneath the epidermis, where they support the skin's structural integrity by generating and organizing the ECM components that maintain skin firmness and elasticity.

The extracellular matrix (ECM) is a complex network of proteins, glycoproteins, and polysaccharides that fills the spaces between cells in tissues, providing both physical support and biochemical cues. In the skin, the ECM acts as a scaffold for cells like fibroblasts and helps regulate processes such as cell migration, growth, and differentiation. The ECM's composition and organization in the dermis influence the skin's properties, such as its ability to stretch, recoil, and resist damage. A well-maintained ECM is essential for skin resilience, wound healing, and its ability to recover from injuries. Disruptions to the fibroblast activity or ECM integrity can result in skin aging, scarring, or conditions like fibrosis.

Sebum is produced by sebaceous glands which are holocrine glands composed of specific cells called sebocytes. Undifferentiated sebocytes located at the periphery of the gland proliferate and some of them regularly begin to differentiate and to produce more and more lipid droplets while migrating toward the center of the gland. The ultimate final stage of sebocyte differentiation is their death which allows the release of the cellular content in the duct of the hair follicle to which the gland is generally connected. This constitutes sebum, mainly composed of neutral lipids, and the amount of sebum produced depends both on sebocyte proliferation and lipid metabolism stimulation.

Acne is a common skin condition that occurs when hair follicles become clogged with oil, dead skin cells, and sometimes bacteria. The features of acne can vary, but they typically include pimples (papules and pustules), blackheads (open comedones), whiteheads (closed comedones), cysts, nodules, inflammation and redness, scarring and oily skin. Pimples are small, raised bumps that are red or inflamed. Pustules contain pus, while papules do not. Blackheads are small, dark spots on the skin caused by clogged hair follicles with a mix of oil and dead skin cells. The dark color is due to oxidation, not dirt. Whiteheads are clogged pores that are closed at the surface, causing white bumps to appear on the skin. Cysts are large, painful, and swollen lumps filled with pus deep beneath the skin's surface. Cystic acne can lead to scarring if untreated. Nodules are firm, painful bumps beneath the surface of the skin that can be larger and deeper than other types of acne. In acne, the infected pores can become swollen, inflamed, and red due to bacteria and irritation. Moreover, severe or untreated acne can lead to scars, which may be raised (hypertrophic) or indented (atrophic). Moreover, excess sebum production is often a cause of acne, leading to an oily appearance of the skin, especially in areas like the forehead, nose, and chin (often called the T-zone).

Acne is most common during puberty but can affect people of all ages. It is typically found on the face, but can also appear on the back, chest, shoulders, and neck. The severity of acne can vary from mild to severe.

The pathogenesis of acne is complex, with the sebaceous gland playing a prominent role. In adulthood the etiopathogenesis of *acne vulgaris* involves a complex interaction between the main factors such as: genetic predisposition; androgenic hormone stimulation leading to an increase in sebaceous secretion; alteration of the lipid composition; follicular hyper keratinization; bacterial colonization mainly by *Cutibacterium acnes* (*C. acnes*) and peri glandular dermal inflammation. This activity results in the formation of sebum plugs, which, combined with other factors, results in the characteristic open (blackheads) and closed (whiteheads) comedones of acne.

Cytokines are regulators produced by host cells in response to infections and immune responses. IL-6, IL-8 are the most well-studied cytokines in acne research. IL-6 and IL-8 are secreted by monocytes, keratinocytes, and sebocytes when stimulated by *C*. *acnes* or substance P and elevated expression levels of IL-6 and IL-8 in acne lesions have been reported. In this regard, IL-6 functions as a proinflammatory cytokine and induces pilosebaceous duct hyperkeratosis, while IL-8 is crucial for attracting neutrophils to the site of inflammation in acne vulgaris, the pilosebaceous unit. The release of lysosomal enzymes by these neutrophils leads to rupture of follicular epithelium and further inflammation.

Skin aging refers to the process by which the skin undergoes physical changes over time due to a combination of internal (genetic) and external (environmental) factors. As we age, the skin naturally loses some of its elasticity, firmness, and youthful appearance. This process typically leads to the following changes: wrinkles and fine lines, loss of firmness, dryness and thinning, age spots, uneven skin tone and skin sensitivity, among others.

The dermal extracellular matrix (ECM) is vital for skin integrity and function, and it is composed mainly by collagen, elastin, fibronectin, proteoglycans, and glycosaminoglycans. Aging alters ECM composition, leading to reduced elasticity and wrinkle formation.

Skin ageing is also associated with a decrease in collagen and delayed wound healing. Ageing causes alteration in inflammatory response as well as cell migration, proliferation, maturation, and scar remodeling; hence having a great influence on effective healing of skin. Ageing alters the mechanisms of wound healing due to a decrease in cell proliferation and migration, deficits in matrix secretion, and a lack of mitogenic growth factors. Each of the cell types involved therein (keratinocytes, fibroblasts, and endothelial cells) shows reduced proliferation and generalized changes in matrix deposition in the wounds of aged organisms.

Fibroblasts and keratinocytes migrate to the site of the wound and start proliferating, marking the next phase of the wound healing process. As the major cellular component of the dermis, fibroblasts play an important role in the wound healing process of the skin.

Glycation is a chemical reaction between the carbonyl groups of reducing sugars and the free amino acid groups of proteins. Advanced Glycation End products (AGEs) are brown substances formed in the late stage of glycation reaction between glucose or other reducing sugars and free amino groups in proteins, nucleic acids or lipids.

Skin interstitial tissue and collagen accelerate ageing under the influence of non-enzymatic browning. Multiple comprehensive studies have found that AGEs strongly affect the dynamic balance of the skin and are a pathogenic factor for skin complications of chronic metabolic diseases, such as diabetic skin ulcers, infections, and non-healing wounds, and are the reason for the frequent occurrence of skin problems in the modern population. Studies have shown that with the increase in the AGE content in the skin, people developed skin problems such as yellowing, browning, poor elasticity, and deeper wrinkles.

AGEs cause collagen to lose its elasticity and resistance, making it rigid. As a result, wrinkles, fine lines and sagging may begin to appear as the skin loses its natural structural support. Another effect of glycation is inflammation. As the process progresses, AGEs begin to interact with other receptors in the skin, which can trigger an inflammatory response, causing dryness or discomfort.

Therefore, the ability to suppress AGE formation is directly related to the anti-glycation activity and consequently leads to anti-aging properties.

Different approaches have been described in the art to minimize the effects of acne and aging on the skin.

For instance, WO2020197669A1 describes a method for managing a skin type selected from acneic skin, acne-prone skin and blemish-prone skin in a subject which comprises a step of topically administering an effective amount of a topical composition comprising an excipient and a specific polylysine dendrimer or a salt thereof to said subject.

EP4382090 A1 describes cosmetical compositions containing at least one Bacillus strain or a fermentation broth and at least one ceramide having dermatological applications, in particular for treating or preventing a disease associated with pathogenic *Cutibacterium* acnes and/or for treating or preventing acnes vulgaris, dermatitis, *Rosaceae* or *Couperose.*

WO2019027167A1 relates to a pharmaceutical composition comprising piperonylic acid as an effective ingredient for delaying skin senescence and regenerating the skin.

EP3655112A1 discloses an intracellular extract of *Salinicoccus hispanicus* that can be used for a moisturizing treatment of the skin or scalp, smoothing the skin, soothing, reducing redness and feelings of tightness and cutaneous discomfort, and to slow down skin aging.

However, there is still a need to provide further cosmetic active ingredients and cosmetic compositions to improve the state of the skin, preferably, human skin. In particular, there is a need to provide further cosmetic active ingredients and cosmetic compositions to confer both an improvement of acne-prone skin and skin rejuvenation.

### Summary of the invention

The present invention relates, in a first aspect, to a ferment extract of a strain of *Bacillus velezensis* species deposited in the Spanish Type Culture Collection under accession number CECT 31027.

In a second aspect, the present invention relates to the use of the ferment extract as disclosed herein for the cosmetic, non-therapeutic treatment and/or care of the skin, mucous membranes, hair and/or nails.

In a third aspect, the present invention relates to the cosmetic use of the ferment extract as disclosed herein for dermal application for improving the state of the skin.

In a fourth aspect, the present invention relates to a method for the cosmetic, non-therapeutic treatment and/or care- of the skin comprising the topical administration to a subject in need thereof of an effective amount of the ferment extract as disclosed herein.

In a fifth aspect, the present invention relates to a cosmetic composition for topical application comprising a cosmetically effective amount of the ferment extract as described herein.

In a sixth aspect, the present invention relates to a cosmetic method for improving the state of the skin, preferably human skin, comprising applying topically to the skin of a subject in need thereof of an effective amount of the cosmetic composition as described herein.

### Brief description of the figures

FIG. 1 schematically shows the sebocyte growth and the stimulation protocol.
FIG. 2 shows the cell viability in human dermal fibroblasts (HDF) and in human sebocytes in the presence of *Bacillus velezensis* extract.
FIG. 3 shows representative images depicting the three channels 4',6-Diamidino-2-phenylindole (DAPI) (nuclei), Fluorescein isothiocyanate (FITC) (neutral lipids) and Tetramethylrhodamine (TRITC) (total lipids), and the merged image showing colocalization of neutral and total lipids.
FIG. 4 shows the total and neutral lipids production by human sebocytes. The quantification of total and neutral lipids is shown in FIG. 4A, while FIG. 4B shows the fluorescent images. Data represents mean ±SD, and statistics were performed by the one-way ANOVA followed by Dunnett's posttest, where *P<0.05 vs linoleic acid.
FIG. 5 shows the production of cytokines IL-6 and IL-8 by human sebocytes. Human sebocytes were stimulated and treated with Linoleic acid (LA) and the Bacillus velezensis ferment extract for 4 days, and IL-6 and IL-8 were quantified. Data represents mean ± SD. Statistics were performed by the one-way ANOVA followed by Dunnett's posttest, where *P<0.05 and ***P<0.001 vs. Linoleic Acid.
FIG. 6 shows the collagen type I and elastin protein levels in HDF. HDFs were stimulated with the *Bacillus velezensis* ferment extract for 24 h and Collagen Type I and Elastin protein levels were quantified. Data represents mean ± SD. Statistic was performed by the one-way ANOVA followed by Dunnett's posttest, where *P<0.05, ***P<0.001 and ****P<0.0001 vs. Control.
FIG. 7 depicts the migration of dermal fibroblasts. HDFs were stimulated with the *Bacillus velezensis* ferment extract for 48 hours and cellular migration was quantified. Data represents mean ± SD. Statistics were performed by the Unpaired t-test, where **P<0.01 vs. Control.
FIG. 8 shows the formation of Advanced Glycation End-products (AGEs) measured in the presence of the *Bacillus velezensis* ferment extract. Data represents mean ± SD. Statistics were performed by the one-way ANOVA followed by Dunnett's posttest, where *P<0.05 vs. Control.

### Detailed description of the invention

The object of the present invention is a ferment extract of a strain of *Bacillus velezensis* species, preparation process, compositions and its uses thereof, in particular its cosmetic use for improving the state of the skin.

In the context of the present invention "skin" is understood to be the layers which comprise it, from the uppermost layer or stratum corneum to the lowermost layer or hypodermis, both inclusive. These layers are composed of different types of cells such as keratinocytes, fibroblasts, melanocytes and/or adipocytes among others. In the context of this invention, the term "skin" includes the scalp. The term "skin" includes human skin.

The term "treatment", as used in the context of the present invention when it is not accompanied by the qualifications "cosmetic" or "non-therapeutic", means the administration of a compound according to the invention to alleviate or cure a disease or disorder, or reduce or eliminate one or more symptoms associated with this disease or disorder, or alleviate or eliminate the physiological consequences of the disease or disorder.

Where the term "treatment" or "care" is accompanied by the qualification "cosmetic", it means that the treatment or care is non-therapeutic and has the aim of improving the aesthetic appearance of the skin. This can be by improving properties of the skin such as, but not restricted to, the level of hydration, elasticity, firmness, shine, tone or texture, which properties affect the cosmetic appearance of the skin.

The term "care" in the context of this specification refers to the maintenance of properties of the skin. The properties of the skin are subject to improvement and maintenance through cosmetic treatment and/or care of the skin both in healthy subjects as well as those who present disorders or conditions of the skin and/or mucous membranes, such as and not restricted to, ulcers and lesions on the skin, psoriasis, dermatitis, rosacea or acne, among others.

The term "prevention", as used in this invention, refers to the ability of the active of the invention to prevent, delay or hinder the appearance or development of a disorder and/or condition or skin property/feature before its appearance.

In the context of the present invention, the term "ageing" refers to the changes experienced by the skin with age (chronoaging) or through exposure to the sun (photoaging) or to environmental agents such as tobacco smoke, extreme climatic conditions of cold, heat, or wind, chemical contaminants or pollutants, and includes all the external visible and/or perceptible changes through touch, such as and not restricted to, the development of discontinuities on the skin such as wrinkles, fine lines, furrows, irregularities or roughness, increase in the size of pores, loss of elasticity, loss of firmness, loss of smoothness, loss of the capacity to recover from deformation, sagging of the skin such as sagging cheeks, the appearance of bags under the eyes or the appearance of a double chin, among others, changes to the color of the skin such as marks, reddening, bags under the eyes or the appearance of hyperpigmented areas such as age spots or freckles among others, anomalous differentiation, hyperkeratinization, elastosis, keratosis, hair loss, orange-peel skin, loss of collagen structure and other histological changes of the stratum corneum, of the dermis, epidermis, vascular system (for example the appearance of spider veins or telangiectasias) or of those tissues close to the skin, among others.

In the context of the present invention, an "extract" means a preparation of a substance obtained from plants, animals, bacteria, fungi or yeast by means of a technical process, and used as an active ingredient.

### Ferment extract

In the first aspect, the present invention relates to a ferment extract from a strain of *Bacillus velezensis* species. Said extract is useful in the non-therapeutic treatment, cosmetic treatment and/or care of the skin, mucous membranes, hair and/or nails. The inventors have found that the extract of the first aspect has good activity as a new cosmetic active ingredient. The ferment extract of the first aspect can be obtained from a strain of *Bacillus velezensis* species. Advantageously, this new cosmetic ingredient comprising the ferment extract from a strain of *Bacillus velezensis* species can be obtained from natural resources and is surprisingly effective in the non-therapeutic, cosmetic treatment and/or care of the skin, mucous membranes, hair and/or nails.

*Bacillus velezensis* is a gram-positive bacteria species with multiple applications and, up to date, more than 1200 genome sequences of *Bacillus velezensis* strains are available in GenBank-NCBI.

Preferably, the strain of *Bacillus velezensis* species is a strain of *Bacillus velezensis* species deposited in the Spanish Type Culture Collection under accession number CECT 31027. This strain was deposited on March 12, 2024 at the Colección Española de Cultivos Tipo (CECT) (Edificio 3 CUE, Parc Cientific Universitat de Valencia, Catedrático Agustin Escardino 9, 46980 Paterna (Valencia), Spain), an institution legally recognized for said purpose according to the Budapest Treaty on the International Recognition of the Deposit of Microorganisms on April 28, 1977.

The strain of *Bacillus velezensis* species deposited in the Spanish Type Culture Collection under accession number CECT 31027 was isolated from the internal tissues of the leaves of *Hyssopus officinalis* in October 2020. *Bacillus velezensis* CECT 31027 is a bacterium that is considered an endophyte. An endophyte is an endosymbiont that lives within a plant, i.e. in *Hyssopus officinalis,* for at least part of its cycle without causing an apparent disease while promoting beneficial effects to the host plant.

The ferment extract from a strain of *Bacillus velezensis* species of the invention is the fermentation product of a strain of *Bacillus velezensis* species. The ferment extract is an extract from a strain of *Bacillus velezensis* species that has been subjected to a fermentation process. Preferably, the extract can be obtained from the cells of the *Bacillus velezensis* species which have been separated out from a fermentation broth or it can be obtained by extraction from the fermentation broth itself, which still contains the cells of the *Bacillus velezensis* species.Thus, ferment extract from a strain of *Bacillus velezensis* species, is referred herein as the ferment extract or simply the extract.

The ferment extract of *Bacillus velezensis* can be obtained or is obtainable by a process which comprises fermenting the strain of the invention in an aqueous culture medium.

The ferment extract of *Bacillus velezensis* can be obtained by a process comprising culturing cells of the bacteria in an aqueous culture medium suitable for the development of the cells in a conventional manner for a person skilled in the art to obtain a biomass of *Bacillus velezensis.*

The fermentation process to obtain the ferment extract of *Bacillus vileness* of the invention (medium and method) has been optimized for lipopeptide production. In a first step a fermentation broth is provided, followed by conventionally stirring and aeration to enable the synthesis and secretion of the desired product in the culture medium, and this is followed by an isolation and purification step of the ferment extract. Fermentation process can be carried out in a medium stirred and aerated at a temperature of between 15°C and 40 °C, typically at about 25°C, with the medium having a pH between 5 and 9, typically around 7.5. The pH can be adjusted if necessary, during fermentation. The duration of the fermentation process is between 2 to 10 days, preferably in one embodiment between 3 to 8 days, and more preferably in another embodiment, between 4 and 7 days.

The steps of isolation and purification of the ferment extract obtained from the fermentation broth can be carried out by methods known to the person skilled in the art, such as centrifugation, disruption and extraction. For example, in a first step, centrifugation can be used to separate the cells from the strain of the *Bacillus velezensis* species from the culture broth; in a second step, disruption can be used to release the intracellular compounds; and in a third step, an extraction of the intracellular compounds can be performed to obtain the ferment extract. The ferment extract may include those intracellular compounds that are soluble in the extraction solvent. The ferment extract can be obtained simply by extraction of the fermentation broth, that is, without centrifugation and disruption steps. However, preferably a disruption step is employed to maximize yield of the desired product.

In the fermentation process of the strain of *Bacillus velezensis* species the aqueous culture medium may contain exogenous sugars, such as and not restricted to, galactose, glucose, mannose, mannitol, sucrose, amygdalin, cellobiose, maltose, starch, glycogen, lactose, mixtures thereof and/or extracts containing mixtures of these sugars, which can be used as a carbon source. In one embodiment, an exogenous supply of sucrose in a concentration from 2 to 40 g/L, or from 5 to 30 g/L, preferably about 22 g/L can be provided.

The aqueous culture medium may comprise additional nitrogen or carbon sources such as yeasts extracts, malt extracts or peptones, with concentrations of each one of these components from 0.1 to 20 g/L, or from 0.5 to 15 g/L. Preferably, the aqueous culture medium may comprise yeast extract, rice peptones and malt extract in a concentrations of about 5 g/L, about 10 g/L, and about 5 g/L, respectively.

The culture medium may also comprise salts, preferably inorganic salts. The salts may be present at a concentration from 1 to 40 g/L, or from 2 to 20 g/L. In one embodiment, inorganic salts are also provided to the fermentation culture medium. These salts are selected among salts which provide the ions selected form the group consisting of, Na⁺, K⁺, NH₄⁺, Ca²⁺, Mg²⁺, PO₄³⁻, SO₄²⁻, Cl⁻, F⁻, CO₃²⁻, NO₃⁻, citrates, and mixtures thereof, or trace elements such as Cu, Mn, Mo, Fe, Sr, B, Br, Si, Al, Li, Zn and mixtures thereof. Preferably, the culture medium comprises sodium nitrate in an amount of about 2.65 g/L.

In particular, the ferment extract may comprise a supernatant and a lysate of the bacterial cells of *Bacillus velezensis.* The ferment extract of the invention may be obtained by culturing the bacterial cells in an aqueous culture medium.

In the context of the present invention, a "lysate" means any extract derived from cells obtained by a method comprising at least an enzymatic hydrolysis, a chemical hydrolysis, an osmotic disruption or a mechanical disruption step of the plasma membrane of a population of said cells. In a particular embodiment the cells are bacterial cells.

Preferably, the supernatant comprises the culture medium.

Even more preferably, the extract of *Bacillus velezensis* comprises a supernatant, a lysate of the bacterial cells (*Bacillus velezensis*) obtained by culturing said cells in a culture medium, and the culture medium.

Preferably, the lysate of the bacterial cells is obtained by mechanical disruption, enzymatic hydrolysis or chemical hydrolysis.

Preferably, the lysate of the *Bacillus velezensis* is obtained by a process comprising mechanical disruption, in particular by increasing the temperature and/or the pressure.

The ferment extract from the *Bacillus velezensis* species strain typically contains peptides, free amino acids, carbohydrates and lipids. In one embodiment, the ferment extract comprises at least 30 ppm of lipopeptides (as measured by High Peformance Liquid Chromatography, HPLC) and at least 500 ppm of peptides having a molecular mass lower than 8 KDa (as measured by Size-Exclusion Chromatography, SEC, and quantified by the Bicinchoninic acid assay, BCA). The ferment extract may further comprise lipopeptides, preferably a combination of surfactin, iturin and fengicine.

In another aspect, the present invention is related to the use of the ferment extract for cosmetic treatment, non-therapeutic treatment and/or care of the skin, mucous membranes, hair and/or nails.

In particular, the ferment extract of the invention may be particularly effective in cosmetic treatment, non-therapeutic treatment and/or care of the skin, including: the smoothing out of or reduction of skin wrinkles; the reduction in volume of a puffy eye or of eye bags; the maintenance or improvement of skin elasticity; the maintenance or improvement of skin resistance, firmness or tensile strength. It has been found that the ferment extract can promote collagen and elastin synthesis. Additionally, said ferment extract may serve to replenish and maintain collagen and elastin levels in aging skin, thus maintaining and improving skin elasticity, skin resistance and/or skin tensile strength. This leads to more toned and firmer skin which has less of a tendency to sag and/or wrinkle. It has also been found that said ferment extract can inhibit the formation of Advance Glycation End products (AGEs). It is believed that this further adds to the ferment extract's ability to help maintain collagen (collagen Type I) levels in aging skin, and, as a result, maintain skin elasticity. Further, this ability to inhibit the formation of AGEs leads to a decrease in the formation of compounds which along with a decrease in elastin is believed to cause skin dullness, and thus use of this cosmetic active can improve skin luminosity. Thus, the present invention also provides for the cosmetic use of said ferment extract for the maintenance or improvement of skin elasticity, skin resistance, skin firmness, skin tensile strength. The present invention is also related to the cosmetic use of said ferment extract for the maintenance or improvement of skin elasticity, skin resistance, skin tensile strength, and the cosmetic use of said ferment extract as a skin firming and toning agent.

The present invention is also related to the use of the ferment extract of the invention in the alleviation and/or prevention of the symptoms or signs of skin aging. Thus, the invention relates to the cosmetic or non-therapeutic use of said ferment extract in the alleviation and/or prevention of the symptoms of skin aging and the cosmetic use of said ferment extract as a skin anti-aging agent. The symptoms or signs of skin aging include: the appearance of skin wrinkles, age spots, the dulling of the skin, loss of skin elasticity, skin resistance, skin firmness and/or skin tensile strength. Thus, the cosmetic treatment and/or care includes: the treatment of skin wrinkles; the smoothing out or reduction in size of skin wrinkles; the maintenance or improvement of skin elasticity, the improvement of skin tone evenness, skin resistance, skin firmness and/or skin tensile strength.

In particular, the present invention relates to a cosmetic use wherein the ferment extract reduces the amount of lipids in sebocytes, inhibits the production of cytokines, promotes collagen production, promotes elastin production, improves fibroblast migration and/or reduces the production of advanced glycation end products. Preferably, the cytokines are inflammatory cytokines, more preferably the cytokines are IL-6 and IL-8.

The present invention also relates to cosmetic or non-therapeutic use of the ferment extract as described herein for topical application for improving the state of the skin.

In particular, improving the state of the skin may be selected from the group consisting of improving the appearance of acne-prone skin, reducing the appearance of oil or pores size on the skin, reversing the effects caused by ageing in the skin, and combinations thereof.

The present invention also relates to a method for cosmetic, non-therapeutic treatment and/or care of the skin, the method comprising the topical administration to a subject in need thereof an effective amount of the ferment extract as disclosed herein.

### Cosmetic active ingredient

In the context of the present description, a "cosmetic active ingredient" means a compound or a set of compounds having a cosmetic effect on the skin, in particular on the skin cells.

The present invention also relates to a cosmetic active ingredient comprising the ferment extract of *Bacillus velezensis* herein described, having a cosmetic effect on the skin, in particular on the skin cells, thus being present in a cosmetic effective amount.

The cosmetic active ingredient of the present invention comprises generally from 0.001 wt% to 20 wt% of the ferment extract of *Bacillus velezensis,* preferably from 0.1 w% to 20%, more preferably from 2 wt% to 10 wt%, even more preferably from 4 wt% to 8 wt, based on the total weight of the cosmetic active ingredient. Thus, in all these concentrations ranges, the ferment extract is present in a cosmetic effective amount.

Preferably, the cosmetic active ingredient comprises the ferment extract of *Bacillus velezensis* and further a cosmetically acceptable excipient selected from the group consisting of; co-solvents, thickeners, additives, and mixtures thereof. A cosmetic excipient is defined herein as inactive ingredients placed alongside active ingredients to provide a cosmetic active or a cosmetic product, for cosmetic use.

The cosmetic active ingredient may comprise a co-solvent. Generally, the co-solvent is selected from the group consisting of 1,3-propanediol, 1,2-propanediol (propylene glycol), polyethylene glycol, glycerin, and mixtures thereof, preferably the co-solvent is a mixture of glycerin and 1,3-propanediol. The content of the co-solvent or the co-solvent mixture is generally in an amount of between 5 wt% and 60 wt%, preferably between 10 wt% and 50 wt%, more preferably between 10 wt% and 40 wt%, even more preferably between 10 wt% and 30 wt% with respect to the total weight of the cosmetic active ingredient.

The cosmetic active ingredient may further comprise an additive. Generally, the additive is selected from the group consisting of pentylene glycol, levulinic acid, glyceryl caprylate, sodium benzoate, potassium benzoate, sodium propionate, potassium sorbate, gluconolactone (δ-lactone of gluconic acid), lactic acid, tartaric acid, and mixtures thereof; preferably the additive is selected from the group consisting of pentylene glycol, levulinic acid, glyceryl caprylate, and mixtures thereof. Preferably, the additive is a mixture of pentylene glycol, levulinic acid, and glyceryl caprylate. Even more preferably, the cosmetic active ingredient comprises glyceryl caprylate in an amount of between 0.01 wt% and 5 wt%, preferably between 0.1 wt% and 0.5 wt%, more preferably between 0.1 wt% and 0.5 wt%, even more preferably between 0.12 wt% and 0.16 wt% with respect to the total amount of the cosmetic active ingredient; the cosmetic active ingredient comprises levulinic acid in an amount of between 0.01 wt% and 5 wt%, preferably between 0.1 wt% and 0.5 wt%, more preferably between 0.1 wt% and 0.5 wt%, even more preferably between 0.3 wt% and 0.4 wt% with respect to the total amount of the cosmetic active ingredient; and the cosmetic active ingredient comprises pentylene glycol in an amount of between 0.01 wt% and 10 wt%, preferably between 0.1 wt% and 5 wt%, more preferably between 2 wt% and 4 wt%, even more preferably between 2.5 wt% and 3.5 wt% with respect to the total amount of the cosmetic active ingredient;

In a preferred embodiment, the cosmetic active ingredient comprises the ferment extract of *Bacillus velezensis,* a co-solvent, preferably 1,3-propanediol and/or glycerin, and as additive, preferably a combination of pentylene glycol, levulinic acid, and glyceryl caprylate.

The present invention also relates to a method for cosmetic, non-therapeutic treatment and/or care of the skin, the method comprising the topical administration to a subject in need thereof of the cosmetic active ingredient as disclosed herein.

### Cosmetic composition

In another aspect of the invention, the ferment extract of the invention and/or the cosmetic active ingredient of the invention can also be incorporated into cosmetic or dermopharmaceutical composition.

Another aspect of the present invention is a cosmetic or a non-therapeutic composition for topical application comprising the ferment extract of the invention and at least one cosmetically acceptable excipient or the cosmetic active ingredient as defined herein and optionally a cosmetically acceptable excipient. The cosmetic composition for topical application comprises a cosmetically effective amount of the ferment extract as described in the present invention. The ferment extract of the invention present in the cosmetic composition can be directly used in the composition or be incorporated as part of the cosmetic active ingredient of the invention.

In an embodiment, the cosmetic composition contains at least one cosmetically acceptable excipient.

In the context of the present invention, "cosmetically acceptable" means that the product(s) or compound(s), which the term describes are suitable for use in contact with tissues (e.g., the skin) without undue toxicity, incompatibility, instability, irritation, allergic response, and the like. This term is not intended to limit the product, which describes to use solely as a cosmetic or as a product for non-therapeutic use.

In an embodiment, the cosmetic composition may comprise at least one cosmetically acceptable excipient selected from the group consisting of: co-solvents, thickeners, adjuvants, additives, and combinations thereof.

In a particular embodiment, the cosmetic composition may comprise at least one cosmetically acceptable excipient selected from the group consisting of; co-solvents, surfactants or emulsifiers, lipid compounds, emollients, consistency factors, thickeners, adjuvants, additives, stabilizers, hydrotropes, preservatives, essences, perfumes, colorants, silicone compounds, fats, waxes, lecithins, phospholipids, UV sun protection factors, film-forming agents, self-tanners, firming agents, and mixtures thereof.

Preferably, the additive is selected from the group consisting of pentylene glycol, levulinic acid, glyceryl caprylate, and combinations thereof.

Preferably, the co-solvent is selected from the group consisting of glycerin, propanediol, and combinations thereof.

A cosmetically acceptable vehicle (or carrier) is a vehicle in which the cosmetic ingredients are dissolved, emulsified, dispersed, or suspended. Said vehicle is selected from among water, a water-miscible non-aqueous vehicle, such as ethanol or isopropanol, and a water-immiscible non-aqueous vehicle, such as, for example, vegetable oil, fatty esters, medium chain triglycerides, or alkanes. Preferably the cosmetic composition includes water as a vehicle.

In an embodiment, the cosmetic composition may further comprise other cosmetically active ingredient. The cosmetically active ingredient may be selected from: skin soothing and healing agents, skin anti-aging agents, skin moisturizing agents, anti-wrinkle agents, skin smoothing agents, antibacterial agents, antifungal agents, antimicrobial agents, anti-inflammatory agents, anti-pruriginous agents, external anesthetic agents, keratolytic agents, free radicals scavengers, anti-seborrheic agents, antidandruff agents, the agents modulating the differentiation, proliferation or pigmentation of the skin and agents accelerating penetration, desquamating agents, depigmenting or pro-pigmenting agents, tightening agents, agents stimulating the synthesis of dermal or epidermal macro molecules and/or preventing their degradation, agents stimulating the proliferation of fibroblasts and/or keratinocytes or stimulating the differentiation of keratinocytes, muscle relaxants; antipollution and/or anti- free radical agents, slimming agents, anticellulite agents, agents acting on the microcirculation, agents acting on the energy metabolism of the cells, cleaning agents, hair conditioning agents, hair styling agents, hair growth promoters, sunscreen and/or sunblock compounds, make-up agents, antiseptic agents, deodorant actives, essential oils, cosmetic astringents, anti-acne agents, anti-caking agents, anti-foaming agents, antioxidants, biological additives, enzymes, enzymatic inhibitors, enzyme-inducing agents, coenzymes, plant extracts, plant derivatives, plant tissue extracts, plant seed extracts, plant oils, botanical extracts, ceramides, peptides, cosmetic biocides, denaturants, astringents compounds, film formers, quaternary derivatives, agents increasing the substantivity, opacifying agents, skin bleaching and lightening agents, skin tanning agents, skin-conditioning agents, skin soothing and/or healing agents, skin treating agents, vitamins and derivatives thereof, peeling agents, moisturizing agents, lignans, UV absorbers, water-soluble sunscreen, antiperspirant, depilatory, fat soluble sunscreen, skin restructuring agent, anti-mycobacterial agents, anti-allergenic agents, anti-irritants, immune system boosting agents, immune system suppressing agents, insect repellents, staining agents, photostabilizing agents, and mixtures thereof.

The physical form of the cosmetic composition for the use according to the invention is not a limiting feature of the invention. Said composition may be in the form of or made into a wide variety of product types that include but are not limited to solid, semisolidsand liquid compositions such as solutions, lotions, creams, gels, sticks, sprays, aerosol, ointments, cleansing liquid washes and solid bars, shampoos, conditioners, dermal injectable compositions, pastes, powders, foams, mousses, serums, liniments, balsams, milks, oil-in water emulsions, water-in-oil emulsions, multiple emulsions (water/oil/water or oil/water/oil), microemulsions, nanoemulsions, dispersions, suspensions, or wipes.

The topical compositions useful in the present invention may be formulated as solutions. Solutions may preferably include an aqueous solvent. Preferably, the % of the cosmetically acceptable aqueous solvent in the solution topical composition is from about 75 wt% to about 95 wt% or from about 75 wt% to about 85 wt% to the total weight of the composition . More preferably, such compositions may contain about 80 wt% solvent, although this may vary dependent upon the formulation. Such aqueous solvents may be selected from the group consisting of ethanol, propylene glycol, polyethylene glycol, mixtures thereof and the like. In an embodiment, the topical composition useful in the present invention may be formulated as a solution containing an emollient. Such composition preferably contains from about 2 wt% to about 50 wt% of an emollient. As used herein, "emollients" refer to materials used for the prevention or relief of dryness, as well as for the protection of the skin.

A type of cosmetic composition may be a lotion, which may be made from a solution. Lotions typically contain from about 1 wt% to about 20 wt% of an emollient and from about 50 wt% to about 90 wt% of water.

Another type of cosmetic product may be a cream. A cream typically comprises from about 5 wt% to about 50 wt% of an emollient and from about 45 wt% to about 85 wt% of water.

Another type of cosmetic product may be an ointment. An ointment may be constituted of a simple base of vegetable oils or semi-solid hydrocarbons. An ointment may contain from about 2 wt% to about 100 wt% of an emollient, and from about 0.1 wt% to about 5 wt% of a thickening agent.

The topical compositions useful in the present invention may also be preferably formulated as emulsions. In an preferred embodiment, the cosmetic composition contains water and a lipophilic phase and is presented in the form of an emulsion or a dispersion, such as, for example, the oil-in-water emulsion (O/W), water-in-oil emulsion (W/O) type, multiple emulsion (W/O/W or O/W/O), or PIT-type emulsion, or microemulsion. If the topical composition is in the form of an emulsion, the emulsion may contain a carrier present in an amount from about 1 wt% to about 10 wt% in total weight of the topical composition. The carrier may contain one or more emulsifiers. Emulsifiers may be non-ionic, anionic, cationic, or amphoteric.

The topical composition useful in the present invention may be formulated as a gel. The topical gel compositions may be aqueous gel compositions, which may contain suitable gelling agents for aqueous gels including, but not limited to, natural gums, acrylic acid and acrylate polymers and copolymers, and cellulose derivatives (e.g., hydroxymethyl cellulose and hydroxypropyl cellulose). The topical gel compositions may be oil gel compositions, Suitable gelling agents for oil gel compositions (such as vegetable oils, esters) include, but are not limited to, waxes, modified silicas, cellulose derivatives, polyamides, polyurethanes, and L-glutamic acid derivatives. The topical gel compositions typically comprise between about 0.1 wt% and 20 wt% of such gelling agents to the total weight of the composition.

The topical composition useful in the present invention may also be formulated into a solid formulation (e.g., a wax-based stick, mascara, soap bar composition, powder, a wipe containing powder, or cosmetotextile).

Further examples of cosmetically acceptable ingredients and vehicles are cited i.e. in the International Cosmetic Ingredient Dictionary and Handbook published by the Personal Care Product Council (PCPC).

### Cosmetic method

Another aspect of the invention is a cosmetic method for treating the skin, preferably human skin, comprising applying topically to the skin of a subject in need thereof of an effective amount of a cosmetic composition comprising the cosmetic composition as disclosed herein.

According to the present invention, the cosmetic method for treating the skin, preferably human skin, said method is suitable for improving the state of the skin and may be selected from the group consisting of; a cosmetic method for improving the appearance of acne-prone skin, reducing the appearance of oil or pores on the skin, reversing the effects caused by ageing on the skin, and combinations thereof.

The cosmetic composition of the invention may be applied topically at least once a day for at least seven days. In particular, the cosmetic composition is applied topically at least twice a day for at least 14, 28 or 56 days, preferably twice a day between 14 and 28 days.

### Examples

### Example 1: Preparation of a ferment extract of Bacillus velezensis

A culture of *Bacillus velezensis* species deposited in the Spanish Type Culture Collection under accession number CECT 31027, whose geographic origin is Spain, was grown up in an aqueous culture medium suitable for their development, comprising yeast extract, comprising a mixture of amino acids, peptides, water soluble vitamins and carbohydrates, in a conventional manner for a person skilled in the art, at about 25-30°C.

In particular, the aqueous culture medium had the following components: sodium chloride, yeast extract, rice peptone, malt extract R2, sodium nitrate and sucrose. The components were dissolved in water at concentrations of 15 g/L, 5 g/L, 10 g/L, 5 g/L, 2.65 g/L and 22 g/L, respectively.

The aqueous culture medium had been sterilized (autoclaved) at 121°C for 20 minutes.
10 L of aqueous culture medium were prepared. In aseptic conditions, 1 mL of *Bacillus velezensis* cryostock was added to 1-2 L of aqueous culture medium in an Erlenmeyer flask. The flasks were incubated at 30°C for 96 hours at 11g.

The aqueous culture medium was subsequently autoclaved and stored at 4°C.

### Example 2: Preparation of a cosmetic active ingredient comprising the Bacillus velezensis ferment extract

To the ferment extract obtained as in Example 1 above, the following excipients were added: pentylene glycol, levulinic acid and glyceryl caprylate, as additives. Moreover, co-solvents glycerin and propanediol were added as well.

In a particular embodiment, the cosmetic active ingredient comprising the *Bacillus velezensis* ferment extract was as follows: 50.26 wt% water, 20.10 wt% glycerin, 20.10 wt% propanediol, 6.03 wt% *Bacillus velezensis* ferment extract, 3.02 wt% pentylene glycol, 0.35 wt% levulinic acid and 0.14 wt% glyceryl caprylate.

The solution was homogenized for 10 minutes and was packaged and stored at room temperature.

This solution hereby obtained is a preferred embodiment of the cosmetic active ingredient as disclosed herein.

The formulation of the cosmetic active ingredient according to the present invention may be as disclosed herein: 40-60% water, 10-30% glycerin, 10-30% propanediol, 4-8% *Bacillus velezensis* ferment extract, pentylene glycol 2.5-3.5%, levulinic acid 0.3-0.4%, glyceryl caprylate 0.12-0.16% of each of the components to the total weight of the cosmetic active ingredient composition.

### Example 3: Cosmetic composition comprising the Bacillus velezensis ferment extract

A cosmetic composition, preferably topical, was prepared comprising the cosmetic active ingredient obtained as in Example 2. The cosmetic composition comprises a mixture of the cosmetic active ingredient of example 2 and excipients such as; co-solvents, thickeners, adjuvants, additives and other cosmetically acceptable excipients.

In a preferred embodiment, the cosmetic composition comprises water, 1,3-propanediol, a mixture of microcrystalline cellulose, sphingomonas ferment extract and cellulose gum (PemuPur^{™} START POLYMER, Lubrizol), a mixture of phenylpropanol, propanediol, caprylyl glycol and tocopherol (Sensiva^{®} PA 40, Ashland), a mixture of Carrageenan and Chondrus Crispus Extract (Satiagum VPC 430, Cargill), a mixture of isostearyl isostearate and *Nelumbo Nucifera* Flower Extract (Lotusense, Provital), *Hyssopus Officinalis* Leaf Oil, Fragrance (Parfum) (Magic Shroom, Luzi), Tocopherol (Covi-ox^{®} T 70C, BASF), the cosmetic active ingredient described hereinabove, CI75810 Maltodextrin (Natpure Col Green LC 717, Sensient) and sodium hydroxide.

Preferably, the cosmetic active ingredient is present in the cosmetic composition in an amount of 2% by weight with respect to the total weight of the cosmetic composition.

The cosmetic composition may preferably comprise water (aqua) as vehicle.

In a preferred embodiment, the cosmetic composition may comprise the following components in the following amount, by weight with respect to the total weight of the cosmetic composition:

**Table 1. Example of a cosmetic composition according to the present invention**

| **Ingredient** | **Name (INCI /PCPC)** | **Trade Name** | **Supplier** | **w/w %** |
|---|---|---|---|---|
| A.1 | Water | | | 89.38 |
| A.2 | Microcrystalline cellulose (and) Sphingomonas ferment extract (and) Cellulose gum | PemuPur^{™} START POLYMER | Lubrizol | 0.50 |
| A.3 | 1,3-propanediol | | | 3.00 |
| A.4 | Phenylpropanol, Propanediol, Caprylyl Glycol, Tocopherol | Sensiva^{®} PA 40 | Ashland | 0.8 |
| A.5 | Carrageenan, Chondrus Crispus Extract | Satiagum VPC 430 | Cargill | 1.50 |
| B.1 | Isostearyl Isostearate, Nelumbo Nucifera Flower Extract | Lotusense | Provital | 2.00 |
| B.2 | *Hyssopus Officinalis* Leaf Oil | | | 0.10 |
| B.3 | Fragance (Parfum) | Magic Shroom | Luzi | 0.50 |
| B.4 | Tocopherol | Covi-ox ^{®} T 70C | BASF | 0.20 |
| C.1 | Aqua, Glycerin, Propanediol, Bacillus Ferment, Pentylene Glycol, Levulinic Acid, Glycerly Caprylate | Formulation according to Example 2 | Provital | 2.00 |
| C.2 | CI 75810, Maltodextrin | Natpure Col Green LC 717 | Sensient | 0.01 |
| C.3 | Water, sodium hydroxide | | | 0.01 |

Moreover, the cosmetic composition described above may be obtained by process comprising the following steps: dispersing PemuPur START^{™} Polymer (ingredient A.2) in water (A.1) using a homogenizer for 10 min; leaving the dispersion for 15 minutes; blending ingredients A.3, A.4 and A.5 together and adding them to the dispersion obtained before under stirring conditions, until a homogeneous gel is formed; mixing ingredients B.1, B.2, B.3 and B.4 together and slowly add this mixture to the homogenous gel obtained before under strong stirring conditions; homogenizing the mixture for 1 minute; adding components C.1 and C.2 to the resulting mixture obtained before; and adjusting the pH to between 5 and 6 with ingredient C.3.

The cosmetic composition as described herein may preferably have a pale green creamy gel appearance, a pH of between 5.0 and 6.0, a viscosity (100 rpm) of 800-1600 mPa·s and/or may be stable for three months at 50°C, 40°C, room temperature and 4°C. The stability studies were performed in a closed container, opening it only to take the corresponding measurements.

The following Examples 4 to 10 refer to *in vitro* assays sought to evaluate the anti-acne effect of the *Bacillus velezensis* ferment extract according to the present invention, by analyzing the production of sebum by sebocytes and the effect on inflammatory cytokines. Moreover, the Examples below also evaluate the well-aging activity of the *Bacillus velezensis* ferment extract according to the present invention, by quantifying the collagen and elastin protein levels in human dermal fibroblasts, the study of human dermal fibroblasts migration and the formation of Advanced Glycation End-products.

Statistical differences were determined by using Graphpad Prism^{®} v10 software (Graphpad Software) on a PC. The alpha nominal level is set at 0.05 in all cases. A p value of < 0.05 was considered significant where * P <0.05, **P<0.01, ***P<0.001 and ****P<0.0001 vs non-stimulated control. One-way ANOVA was used for experiments comparing three or more groups, while unpaired t test was used for tests comparing two groups.

### Example 4: Sebocyte culture and incubation with Bacillus velezensis

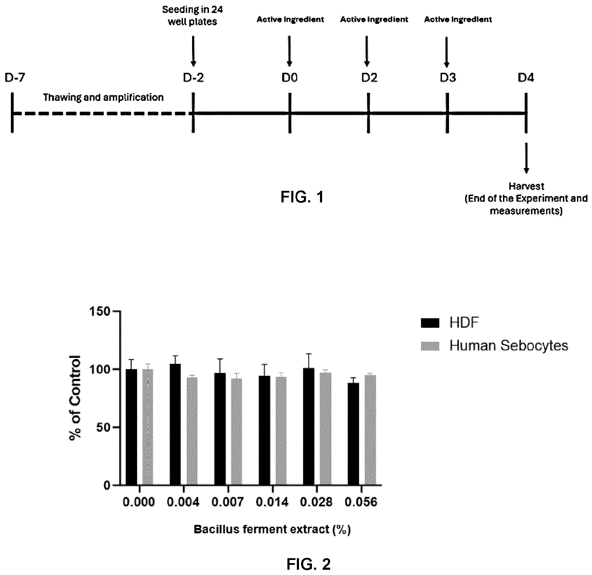

After cell thawing and expansion during 5 days (from day-7 to day-2, as shown in FIG. 1), human sebocytes from a 50-yeard old Caucasian woman were grown in Seb4Gln medium in 24-well plates coated with fibronectin. Two days after seeding, the sebocytes of the previous step were incubated with Bacillus velezensis ferment extract at days 0, 2 and 3. The concentration of the ferment extract tested was 0.028% and 0.056%. Harvesting was performed on day 4 and samples were processed for each of the assays (FIG. 1). Of note, as an inducer of sebum production, and since linoleic acid is directly involved in the sebaceous lipid synthesis, mM linoleic acid was added to sebocytes having the Bacillus velezensis ferment extract or to sebocytes without ferment extract (control samples) at a final concentration of 0.1 mM.

### Example 5: Non-toxicity of the Bacillus velezensis ferment extract in human dermal fibroblasts and human sebocytes

When studying human sebocytes, cell viability was analyzed with the Alamar Blue test. This test is used to investigate the overall health and metabolic activity of cells and tissues in culture. The Alamar Blue reagent is added to the cultures and if the cells are healthy/active the reagent is converted to a fluorescent reagent detectable at a particular wavelength. The product is detected within the media surrounding the cells / tissue.

Culture supernatants were removed and replaced by a new medium containing 10% Alamar Blue. Then, after 1 hour of incubation, culture supernatant containing Alamar Blue was removed and fluorescence (Excitation 570nm-Emission 585nm) was assessed with the TECAN SPARK^{®} fluorometer.

For testing the viability on Human Dermal Fibroblasts (HDF), the ATP (Adenosin triphosphate) assay was used. The amount of ATP is directly proportional to the number of cells present in culture. Therefore, ATP measurement is used as a homogeneous method to determine the number of viable cells. In the present study the luminescent reagent CellTiter-Glo^{®} from Promega^{®} was used. Cells were seeded per well in 96-well opaque plates. After 24 h, subconfluent cells were stimulated for 24 h with increasing concentrations of the *Bacillus velezensis* ferment extract. The concentrations of *Bacillus velezensis* ferment extract tested were 0.004%, 0.007%, 0.014%, 0.028% and 0.056% with a final volume of 100 µl. All conditions were assayed in triplicate. The ATP measurement was then performed according to the manufacturer's instructions. In brief, 100 µl of reagent previously prepared was added per well. After rotating for 2 minutes to induce cell lysis and incubating 10 minutes at room temperature, luminescence was registered using a luminometer (GloMax^{®} Navigator System, Promega).

The average of Relative Light Units (RLU) recorded in triplicates was blanked to wells without cells and viability was calculated as % over non-stimulated control [(RLU test / RLU control) *100].

The inventors sought to understand whether at the doses of 0.004%, 0.007%, 0.014%, 0.028% and 0.056%, the *Bacillus velezensis* ferment extract showed any effect on cell viability. Of note, since LA is used in later experiments to induce sebum production, LA was added alone and together with *Bacillus velezensis* ferment extract when studying viability in sebocytes. Similarly, and to match later experimental conditions, HDF's were stimulated for 1 day, while sebocytes were stimulated for 4 days, as described herein.

As depicted in FIG. 2 there was no cytotoxicity effect found at any of the concentrations in both cell types.

### Example 6: Reduction of total and neutral lipids in human sebocytes by the Bacillus velezensis extract

The inventors sought to analyze whether *Bacillus velezensis* ferment extract had an action in the production of sebum by sebocytes.

Sebocytes were fixed in formaldehyde 4 % w/v (Sigma-Aldrich) and stained with 50µg/mL Hoechst and 7 µg/mL Nile Red (Sigma-Aldrich).

The Fluorescence intensity (FI) produced was measured with a fluorometer (TECAN SPARK^{®}) with the following excitation and emission wavelengths:
1. Nuclei (Hoechst): excitation 356 nm - emission 465 nm
2. Neutral lipids (Nile Red): excitation 475 nm - emission 530 nm
3. Total lipids (Nile Red): excitation 520 nm - emission 625 nm

All conditions were run in triplicate and at least 3 readings across the well were recorded.

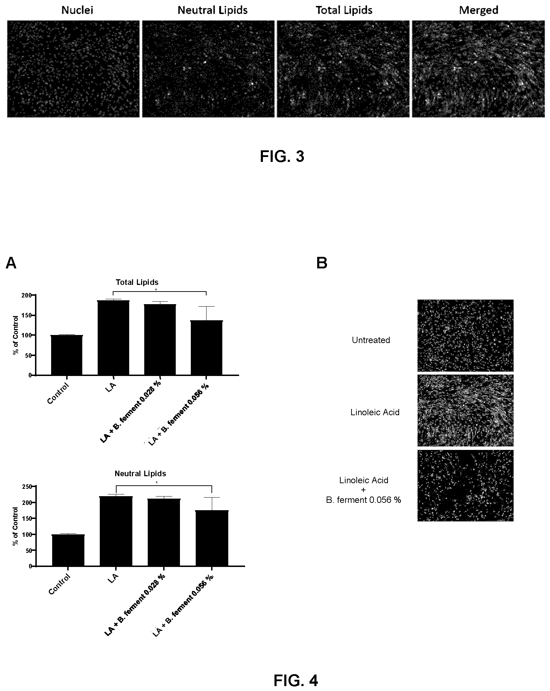

Microscopy images were captured on a fluorescence microscope (Eclipse Ti, Nikon) on standard capturing conditions for DAPI (nuclei, blue), FITC (neutral lipids, green) and TRITC (total lipids, red), where merging showed colocalization of both FITC and TRITC in yellow. FIG. 3 shows a representative image depicting the three channels for nuclei, neutral lipids and total lipids.

In FIG. 4 it is shown that LA (linoleic acid) elicits a strong increase in both total and neutral lipids. Interestingly, this increase is reduced by the *Bacillus velezensis* ferment extract following a dose-response up to *Bacillus velezensis* ferment extract concentration of about 0.056 % by weight, which significantly reduced the production of total and neutral lipids by 27% and 21%, respectively.

Since neutral lipids always colocalize with total lipids, the yellow color of the merging showed the overlap therebetween (total lipids in red, neutral lipids in green).

These results let the inventors conclude that the *Bacillus velezensis* ferment extract and compositions thereof reduce the sebum production by sebocytes, thus helping to reduce the appearance of acne.

### Example 7: Inhibition of the production of cytokines IL-6 and IL-8 by the Bacillus velezensis ferment extract

The inventors sought to analyze whether the *Bacillus velezensis* ferment extract had an action in the inhibition of cytokines IL-6 and IL-8 measured in the sebocyte supernatants as described herein. LA is known to induce the secretion of pro-inflammatory cytokines IL-6 and 8.

Levels of both IL-6 and IL-8 were measured with the ELISA kits DuoSet for Human IL-6 and IL-8, respectively, from R&D Systems, in the supernatants harvested at day 4 following the protocol mentioned herein, following the manufacturer instructions. All conditions were assayed in triplicate.

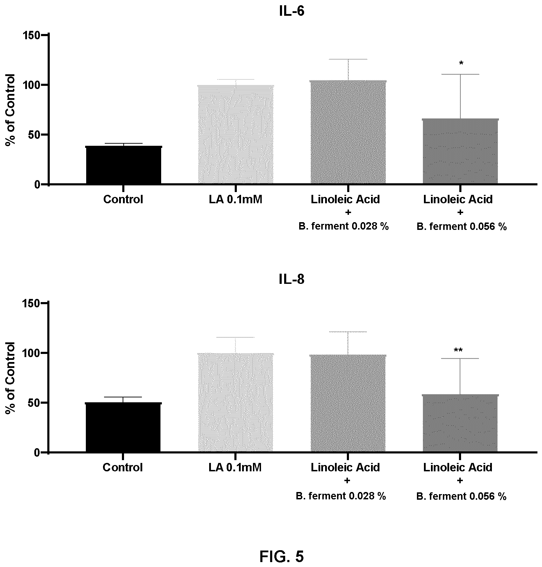

In FIG. 5 it is shown that the *Bacillus velezensis* ferment extract at a concentration of 0.056% by weight significantly reduced the production of both cytokines IL-6 and IL-8 by 33.5% and 41.36% in the presence of LA.

These results indicate that the *Bacillus velezensis* ferment extract at a concentration of 0.056% significantly reduces the levels of both IL-6 and IL-8, thus helping to reduce the inflammatory factors involved in the acne condition.

### Example 8: Increase of collagen type I and elastin in HDFs by the Bacillus velezensis ferment extract

The *Bacillus velezensis* ferment extract effect on Collagen Type I and Elastin production in fibroblasts was analyzed.

HDFs were grown in 6-well plates and stimulated with the *Bacillus velezensis* ferment extract at 0.007% and 0.014% for 24 h. The supernatants were then harvested and stored at -20°C to measure collagen afterwards, while cells were processed to measure the Elastin content.

Collagen Type I was determined in supernatants with the Human Pro-Collagen I alpha 1 DuoSet ELISA from R&D Systems following manufacturer's instructions. After removing the supernatants, the Elastin content was measured with the commercially available kit Fastin (Biocolor Ltd) following the manufacturer instructions. Both Collagen and Elastin levels were normalized to the protein content determined by the Pierce^{™} BCA Protein Assay Kit (Thermo Fisher Scientific) following the manufacturer instructions. All conditions were assayed in triplicate.

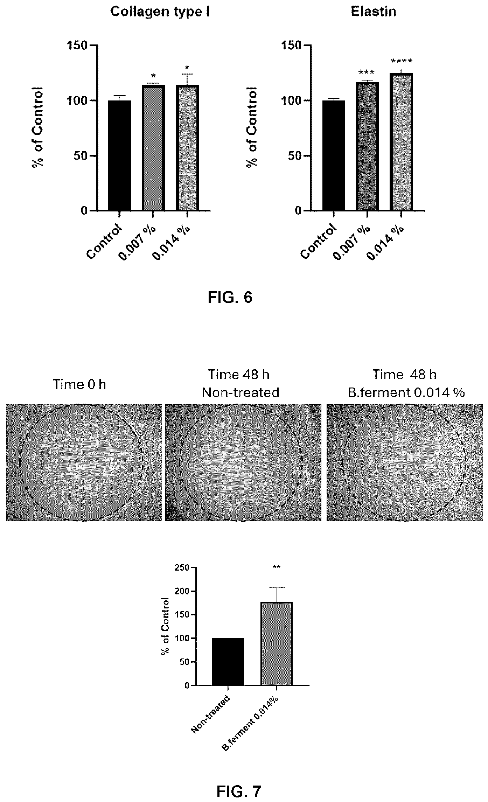

As shown in FIG. 6, it was found that the *Bacillus velezensis* ferment extract significantly increased collagen I (up to 14%) and elastin (up to 25%) levels, at doses previously found not to be toxic in HDF.

### Example 9: Improve of fibroblast migration in HDF by the Bacillus velezensis ferment extract

To further analyze the potential anti-aging effect of the cosmetic active ingredient, a migration test on HDF incubated with the active at 0.014% was performed.

Cell migration was determined using the Oris^{™} Cell Migration Assay (Platypus Technologies, Madison, WI, USA), according to the manufacturer's instructions. This assay involves measuring the migration of cells into an exclusion zone and is superior to the scratch assay, as it does not involve injury to the cells and shows improved reproducibility.

Cells are grown to reach confluency within 24 h in each well of a 96-well plate, which had an Oris^{™} cell seeding stopper in situ to restrict cell seeding to the outer regions of the well. Stoppers for the experimental wells are removed to create a detection zone of 2 mm diameter into which cells could migrate. Cells were thereafter incubated with the control and the *Bacillus velezensis* ferment extract at 0.014% during 48 h at 37°C in 5% CO2, to allow time for migration. After this incubation period, cells migrating into the detection zone are photographed with a microscope (DMi1; Leica Microsystems) and a 5x lens, and quantified using an ImageJ (NIH, Bethesda) plugin based on the concept "confluency". Total area occupied by migrating cells into the detection zone was quantified and expressed as % of control.

The results show that after 48 h, the addition of the *Bacillus velezensis* ferment extract to HDF significantly increased cell migration, to 77% with respect to the control, as shown in FIG. 7.

These results indicate a consistent effect on wound healing properties in the migration test thus revealing that the *Bacillus velezensis* ferment extract is a potential active ingredient for the rejuvenation of the skin.

### Example 10: Reduction of AGEs content by the Bacillus ferment extract

A solution of 1 mg/ml BSA with 200 mM fructose in DPBS was incubated for 7 days at 37°C in the presence of the *Bacillus velezensis* ferment extract at 0.00175% and 0.0035%. After incubation, they were transferred to opaque 96-well plate with clear bottom to quantitatively assess the formation of fluorescent AGEs using a SpectraMax M2 Microplate Reader (Molecular Devices, Sunnyvale) at excitation and emission wavelengths of 355 nm and 460 nm, respectively. % inhibition was calculated as follows:
% inhibition = [1 - (F test) / (F control)] × 100; where "F test" stands for fluorescence of the active at a given concentration, and "F control" for fluorescence without cosmetic active ingredient.

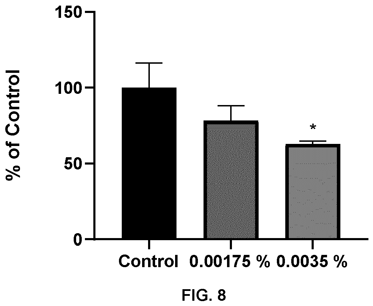

As shown in FIG. 8, AGEs were reduced in a dose-response manner by the *Bacillus velezensis* ferment extract by 37% and 22% vs. control at concentration of 0.0035% and 0.00175% by weight, respectively, being statistically significant only at higher concentration.

These results indicate that the *Bacillus velezensis* ferment extract reduces the production of AGEs, thus contributing to delay the progress of skin aging and promoting the health of cells and tissues.

Therefore, as shown hereinabove, the *Bacillus velezensis* ferment extract proves to be an effective application conferring both anti-acné effect and anti-aging activity on the skin.

The results above show that the *Bacillus velezensis* ferment extract exhibits anti-acne effect (sebum-reducing action and IL-6 and IL-8 cytokines reduction) as demonstrated in cultured primary human sebocytes.

Moreover, the *Bacillus velezensis* ferment extract increased Collagen type I and Elastin in HDF, improved fibroblast migration and is able to reduce AGEs, thus indicating an anti-aging activity.

## Claims

1. A ferment extract of a strain of *Bacillus velezensis* species deposited in the Spanish Type Culture Collection under accession number CECT 31027.

2. The ferment extract according to claim 1, obtainable by fermenting the strain of claim 1 in an aqueous culture medium.

3. The ferment extract according to claim 1 or 2, wherein the ferment extract comprises a supernatant and a lysate of bacterial cells of *Bacillus velezensis.*

4. Use of the ferment extract according to any one of claims 1 to 3 for a cosmetic, non-therapeutic treatment and/or care of the skin, mucous membranes, hair and/or nails.

5. The use of the ferment extract according to claim 4, wherein said use involves the reduction of the amount of lipids in sebocytes, the inhibition of the production of cytokines, the promotion of collagen production, the promotion of elastin production, the improvement of fibroblast migration and/or the reduction in the production of advanced glycation end products.

6. A cosmetic active ingredient comprising the ferment extract according to any one of claims 1 to 3 and at least a cosmetically acceptable excipient, wherein the ferment extract according to claims 1-3 is present in a cosmetically effective amount.

7. A cosmetic or non-therapeutic use of the ferment extract according to any one of claims 1 to 3 or of the cosmetic active ingredient of claim 6 for topical application, wherein the use involves improving the state of the skin.

8. The use according to claim 7, **characterized in that** the use is selected from the group consisting of improving the appearance of acne-prone skin, reducing the appearance of oil or pores on the skin, reversing the effects caused by ageing on the skin, treatment of skin wrinkles, treatment of skin firmness of skin elasticity, treatment of skin resistance, treatment of skin tensile strength and combinations thereof.

9. A cosmetic composition for topical application comprising a cosmetically effective amount of the ferment extract according to any one of claims 1 to 3 and at least one cosmetically accepted excipient, or comprising the cosmetic active ingredient according to claim 6 and at least one cosmetically acceptable excipient.

10. The cosmetic composition according to claim 9, wherein the cosmetically acceptable excipient is selected from co-solvents, thickeners, adjuvants, additives, and combinations thereof.

11. The cosmetic composition according to claim 10, wherein the additive is selected from the group consisting of pentylene glycol, levulinic acid, glyceryl caprylate, and combinations thereof and/or wherein the co-solvent is propanediol, water and/or glycerin.

12. A method for the cosmetic, non-therapeutic treatment and/or care of the skin comprising the topical administration to a subject in need thereof of an effective amount of the ferment extract according to any one of claims 1 to 3, or the cosmetic active ingredient of claim 6, or/and the cosmetic compositions of any one of claims 9 to 11.

13. A cosmetic method for improving the state of the skin, preferably human skin, comprising applying topically to the skin of a subject in need thereof of an effective amount of the ferment extract according to any one of claims 1 to 3, the cosmetic active ingredient of claim 6, or/and the cosmetic compositions of any one of claims 9 to 11.

14. The cosmetic method according to claim 13, **characterized in that** the method for improving the state of the skin is selected from the methods selected from the group consisting of; improving the appearance of acne-prone skin, reducing the appearance of oil or pores on the skin, reversing the effects caused by ageing on the skin, and combinations thereof.

15. The cosmetic method according to claims 13 or 14, **characterized in that** the cosmetic composition according to any one of claims 9 to 11 is applied at least once a day for at least fourteen days.
